# EUROPEAN PATENT SPECIFICATION

(11) **EP 1 682 122 B1**
(45) Date of publication and mention of the grant of the patent: **19.03.2008**
(21) Application number: 04797529.7
(22) Date of filing: 02.11.2004
(51) Int. Cl.: A61K 31/41, A61K 31/4965, A61K 31/549, A61K 45/06, A61P 3/10, A61P 5/00, A61P 5/18, A61P 5/48, A61P 5/50, A61P 9/00, A61P 9/02, A61P 9/04, A61P 9/10, A61P 27/06, A61P 43/00

(54) **COMBINATIONS OF VALSARTAN, AMILORIDE OR TRIAMTERINE, AND A DIURETIC**
KOMBINATIONEN AUS VALSARTAN, AMILORID ODER TRIAMTERIN UND EINEM DIURETIKUM
COMBINAISONS DU VALSARTAN, D'AMILORIDE OU DE TRIAMTERINE, ET D'UN DIURETIQUE

(30) Priority: 03.11.2003 GB 0325605
(43) Date of publication of application: 26.07.2006
(73) Proprietor: Novartis AG, 4056 Basel (CH); Novartis Pharma GmbH, 1230 Wien (AT)
(72) Inventor: VASELLA, Daniel, Lucius, Novartis International AG, CH-4056 Basel (CH)
(74) Representative: Crawley, Patrick Edward
(86) International application number: PCT/EP2004/012386
(87) International publication number: WO 2005/049013

(56) References cited:
- WO-A-01/28548
- WO-A-02/40007
- WO-A-03/097045
- US-A1- 2004 087 484
- DÜSING, R; LEHNERT, H.: "Diabetogenic effect of antihypertensive treatment: primum nil nocere" NEPHROL DIAL TRANSPLANT, vol. 19, no. 3, March 2004 (2004-03), pages 531-534, XP002317306

## Description

The invention relates to a combination, such as a combined preparation or pharmaceutical composition, respectively, comprising
(i) valsartan or a pharmaceutically acceptable salt thereof, and
(ii) amiloride or triameterine a pharmaceutically acceptable salt thereof and
(iii) a further diurectic, such as a thiazide diuretic, or a pharmaceutically acceptable salt thereof.

AT₁-receptor antagonists (also called angiotensin II receptor antagonists or ARBs) are understood to be those active ingredients that bind to the AT₁-receptor subtype of angiotensin II receptor but do not result in activation of the receptor. As a consequence of the inhibition of the AT₁ receptor, these antagonists can, for example, be employed as antihypertensives or for treating congestive heart failure.

The class of AT₁ receptor antagonists comprises compounds having differing structural features, essentially preferred are the non-peptidic ones. For example, mention may be made of the compounds that are selected from the group consisting of valsartan (cf. EP 443983), losartan (cf. EP253310), candesartan (cf. EP 459136), eprosartan (cf. EP 403159), irbesartan (cf. EP454511), olmesartan (cf. EP 503785), tasosartan (cf. EP539086), telmisartan (cf. EP 522314), the compound with the designation E-1477 of the following formula 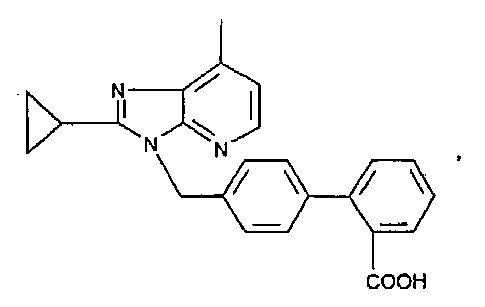 the compound with the designation SC-52458 of the following formula 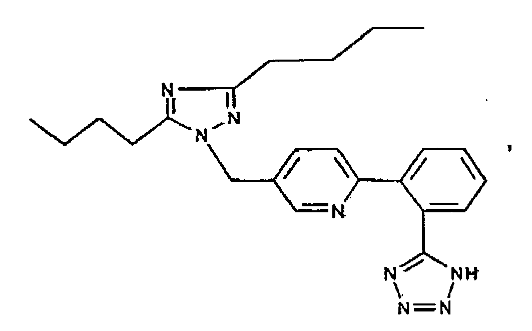 and the compound with the designation the compound ZD-8731 of the following formula 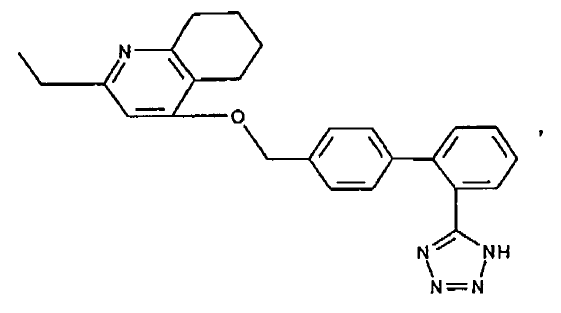 or, in each case, a pharmaceutically acceptable salt thereof.

Preferred AT₁-receptor antagonist are those agents that have been marketed, most preferred is valsartan or a pharmaceutically acceptable salt thereof.

A preferred pharmaceutical acceptable salt of amiloride is the hydrochloride. Amiloiride hydrochloride is the most preferred component (ii).

A thiazide diuretic is, for example, selected from the group consisting of chlorothiazide, hydrochlorothiazide, methylclothiazide, and chlorothalidon. Most preferred is hydrochlorothiazide.

Preferred are combinations, such as a combined preparations or pharmaceutical compositions, respectively, comprising
(i) valsartan or a pharmaceutically acceptable salt thereof and
(ii) amiloride or a pharmaceutically acceptable salt thereof and
(iii) hydrochlorothiazide or a pharmaceutically acceptable salt thereof.

Preferred are combinations, such as a combined preparations or pharmaceutical compositions, respectively, comprising
(i) valsartan or a pharmaceutically acceptable salt thereof; and
(ii) amiloride hydrochloride; and
(iii) hydrochlorothiazide.

The structure of the active agents identified by generic or tradenames may be taken from the actual edition of the standard compendium "The Merck Index" or from databases, e.g. Life Cycle Patents International (e.g. IMS World Publications). Any person skilled in the art is fully enabled to identify the active agents and, based on these references, likewise enabled to manufacture and test the pharmaceutical indications and properties in standard test models, both In vitro and in vivo.

The corresponding active ingredients or a pharmaceutically acceptable salts thereof may also be used In form of a solvate, such as a hydrate or including other solvents, used for crystallization.

The compounds to be combined can be present as pharmaceutically acceptable salts. If these compounds have, for example, at least one basic center, they can form acid addition salts. Corresponding acid addition salts can also be formed having, if desired, an additionally present basic center. The compounds having an acid group (for example COOH) can also form salts with bases.

The diuretics amiloride or triameterine or, in each case, a pharmaceutically acceptable salt thereof block the Na+ channels in the late distal tubules and collecting ducts by increasing the loss of sodium and chloride ions while reducing the excretion of potassium. It is known that the thiazide diuretics reduce the re-absorption of electrolytes from renal tubules, thereby increasing the excretion of sodium and chloride ions and consequently of water. The excretion of potassium is also increased by administering e.g. hydrochlorothiazide. The combination of amiloride, especially the hydrochloride thereof, or triameterine, respectively, and a thiazide diuretic, for example, hydrochlorothiazide, increases the excretion of sodium and chloride ions while diminishing the kaliuretic effects.

All the more surprising is the experimental finding that the combined administration of the combination of
(i) valsartan or a pharmaceutically acceptable salt thereof, and
(ii) amiloride or a pharmaceutically acceptable salt thereof and
(iii) a further diuretic or a pharmaceutically acceptable salt results not only in a beneficial, especially potentiation, preferably a synergistic, therapeutic effect, but also in additional benefits resulting from the combined treatment and further surprising beneficial effects compared to a monotherapy applying only one of the pharmaceutically active compounds used in the combinations disclosed herein.

In particular, all the more surprising is the experimental finding that the combination of the present invention results not only in a beneficial, especially a potentiation, preferably synergistic, therapeutic effect but also in additional benefits resulting from combined treatment such as a surprising prolongation of efficacy, a broader variety of therapeutic treatment and surprising beneficial effects on diseases and conditions as specified hereinafter.

Furthermore, a surprising effect of the combination of the present invention is the fact that a higher blood pressure lowering with lower dose of every component of the three therapy.
- Better potassium handling and homeostasis.
Better protection of the myocardium because of the haemodynamic effects of the three components and the protective effect of valsartan and amiloride. In fact, valsartan by blocking the detrimental actions of AT II on myocardial perfusion and remodeling post myocardial ischemia and necrosis and amiloride by blocking Na+/H+ exchanger that play a role in ischemia-reperfusion injury can protect the myocardium to a high extent in repetitive ischemia and acute myocardial infarction.

It can be shown by established test models and especially those test models described herein that the combination of the therapeutic agents selected from the group consisting of (i) to (iii) results in a more effective prevention or preferably treatment of diseases specified in the following. In particular, it can be shown by established test models and especially those test models described herein that the combination of the present invention results in a more effective prevention or preferably treatment of diseases specified hereinafter.

If taken simultaneously, this results not only in a further enhanced beneficial, especially a synergistic, therapeutic effect, but also in additional benefits resulting from the simultaneous treatment such as a surprising prolongation of efficacy, a broader variety of therapeutic treatment and surprising beneficial effects, e.g. less increase of weight, on diseases and conditions associated with diabetes mellitus, for a number of combinations as described herein. Moreover, for a human patient, especially for elderly people, it is more convenient and easier to remember to take two tablets at the same time, e.g. before a meal, than staggered in time, i.e. according to a more complicated treatment schedule. More preferably, both active ingredients are administered as a fixed combination, i.e. as a single tablet, in all cases desribed herein. Taking a single tablet is even easier to handle than taking two tablets at the same time. Furthermore, the packaging can be accomplished with less effort.

The term "synergistic" as used herein means that the effect achieved with the methods and compositions of the present invention is greater than the sum of the effects that result from methods and compositions comprising the active ingredients of this invention separately.

The person skilled in the pertinent art is fully enabled to select a relevant and standard animal test model to prove the hereinbefore and hereinafter indicated therapeutic indications and beneficial effects.

The pharmaceutical activities as effected by administration of representatives of the class of AT₁-receptor antagonists or diuretics, respectively, or of the combination of active agents used according to the present invention can be demonstrated e.g. by using corresponding pharmacological models known in the pertinent art. The person skilled in the pertinent art is fully enabled to select a relevant animal test model to prove the hereinbefore and hereinafter indicated therapeutic indications and beneficial effects.

The beneficial effects on blood pressure can, for example, be demonstrated in the test model as disclosed in R.L. Webb et al., in J. Hypertension, 16:843-852, 1998.

### Methods:

The combination according to the present invention comprising the compound of formula (I) or a pharmaceutically acceptable salt thereof can be administered by various routes of administration but are tested in this example using a continuous infusion via subcutaneously-implanted osmotic minipumps. Each agent can be tested over a wide-range of dosages to determine the optimal drug level for each agent in combination to elicit the maximal response. For these studies, it is preferred to use treatment groups consisting of at least 6 animals per group. Each study is best performed in which the effects of the combination treatment group are determined at the same time as the individual components are evaluated. Although drug effects may be observed with acute administration (such as 1 day), it is preferable to observe responses in a chronic setting as shown below in which experiments were done over a two to three week observation period. The long-term study is of sufficient duration to allow for the full development of compensatory responses to occur and therefore, the observed effect will most likely depict the actual responses of the test system representing sustained or persistent effects. The effects on blood pressure depicted below represent a synergistic antihypertensive effect when the two agents are used in combination.

### Statistical Analysis:

The combination therapy can be compared to that of the monotherapy groups by determining the maximum change in blood pressure or the area under the curve (AUC) for change in blood pressure over time in each of the treatment groups. All values are represented as the group mean ± SEM. Statistical significance is obtained when p < 0.05. The AUC values for each of the treatment groups can be compared statistically using a one-way ANOVA followed by the appropriate post-hoc analysis, for example by performing a Tukey's test.

### Results:

Blood pressure can be reduced to a similar degree using lower dosages of each of the components when given in combination than when the individual monotherapies are administered. An additional unexpected finding is that the blood pressure can be lowered to a greater extent with the combination than when the individual compound of formulat (I) or a pharmaceutically acceptable salt thereof is given alone at a higher dosage.

These beneficial effects can, for example, be demonstrated in the test model as disclosed by G. Jeremic et al. in J. Cardovasc. Pharmacol. 27:347-354, 1996.

For example, the valuable potential of the combination of the present invention for the prevention and treatment of myocardial infarction (including the post-myocardial infarction indication to delay the progression to congestive heart failure) can be found using the following test model.

### Study design

In the study to be performed, permanent coronary artery occlusion (CAO) in rats is used as a model of acute myocardial infarction. The experiments are carried out with 5 treatment groups characterized by following features:
- sham-operated animals
- CAO + vehicle
- CAO + valsartan (val) or a pharmaceutically accetable salt, thereof,
- CAO + amiloride (ami),
- CAO + hydrochlorothiazide (HCTZ);
- CAO + valsartan or a pharmaceutically accetable salt thereof, + amiloride + hydrochlorothiazide.

During the study following variables are measured:
- infarct size
- LV chamber volume
- interstitial and perivascular collagen density in spared LV myocardium
- COL-I and COL-III protein content in spared LV myocardium by Western blot
- cardiomyocytes cross-sectional area and length in sections of LV myocardium
- plasma concentrations of renin and aldosterone
- urine concentration of sodium, potassium and aldosterone
- blood pressure in conscious animals
- LV and carotid blood pressure in anesthetized animals.

### Methodology

**Infarct size:** Six µm-thick transverse histological sections of the left ventricle are stained with nitroblue tetrazolium and acquired by a B/W XC-77CE CCD video camera (Sony). The resulting image is processed on a KS 300 image analysis system (Carl Zeiss Vision) using a software specifically developed (Porzio *et al.,* 1995). A single operator blinded to treatment interactively defines the boundaries of the interventricular septum, and the infarcted area on each section is semiautomatically identified as the area of unstained ventricular tissue. The software automatically calculates for each component of the ventricular section defined as the chamber, septum, infarcted area, infarcted LV wall and viable LV wall, a set of geometric parameters (Porzio *et al*., 1995).

**Histology:** Hearts are fixed in situ, by retrograde perfusion with buffered 4% formaldehyde after arrest in diastole by i.v. injection of 0.5 M KCl. After fixation, the left ventricle (LV) and the free wall of the right ventricle are separately weighed; LV longer diameter is measured with a caliper. LV histological sections are stained with hematoxylin & eosin for qualitative examination and to quantify cardiomyocytes cross-sectional area with a semi-automated image analysis routine. Interstitial collagen deposition in LV is evaluated on Sirius red stained sections with a semi-automated image analysis routine (Masson *et al.,* 1998).

**Collagen content in LV spared myocardium:** LV tissue in the spared myocardium is homogenized, subjected to PAGE-SDS electrophoresis and electroblotted onto nitrocellulose membrane. The blots are exposed to primary antibodies, i.e. rabbit anti-rat collagen type I or type III antiserum (Chemicon). The primary antibodies are recognized by secondary antibodies conjugated to alkaline phosphatase (for colagen type I) or peroxidase (collagen type III).

**Left ventricular chamber volume:** LV chamber volume is determined in hearts arrested in diastole (KCI) and fixed in formalin under a hydrostatic pressure equivalent to the measured LV end-diastolic pressure. A metric rod is inserted into the LV to measure LV inner length. The transverse diameters of the LV chamber are measured in two 1-mm thick transverse sections near to the base and the apex of the ventricle (Jeremic *et al.,* 1996). The chamber volume is computed from an equation integrating transverse diameters and inner length.

**Systemic and Left ventricular hemodynamics:** A microtip pressure transducer (Millar SPC-320) connected to a recorder (Windograf, Gould Electronics) is inserted into the right carotid artery to record systolic and diastolic blood pressures. The pressure transducer is advanced into the LV to measure LV systolic (LVSP) and end-diastolic (LVEDP) pressures, the first derivative of LV pressure over time (+dP/dt) and heart rate.

**Non-invasive blood pressure:** Systolic blood pressure and heart rate are measured by the tail-cuff method (Letica LE 5002) in conscious rats.

**Urine electrolytes, hormones:** Rats are individually housed in metabolic cages and 24-h urine collected on 1 ml HCl 6N. Water intake is measured. Urine catecholamines are extracted on Bondelut C₁₈ columns (Varian), separated by HPLC (Apex-II C18, 3 µm, 50x4.5 mm analytical column, Jones Chromatography) and quantified with an electrochemical detector (Coulochem II, ESA) (Goldstein *et al.,* 1981). Plasma and urine aldosterone, and plasma angiotensin II are determined with specific radioimmunoassays (Aldoctk-2, DiaSorin and Angiotensin II, Nichols Diagnostics). Urine sodium and potassium are measured by flame photometry.

### Sample size

10 animals analyzable in each treatment groups are sufficient to detect biologically significant differences. Only rats with an infarct size of at least 10% of the LV section area are included in the final analysis.

Endothelial dysfunction is being acknowledged as a critical factor in vascular diseases. The endothelium plays a bimodal role as the source of various hormones or by-products with opposing effects: vasodilation and vasoconstriction, inhibition or promotion of growth, fibrinolysis or thrombogenesis, production of anti-oxidants or oxidising agents. Genetically predisposed hypertensive animals with endothelial dysfunction constitute a valid model for assessing the efficacy of a cardiovascular therapy.

Endothelial disfunction is characterized by, for example, increased oxidative stress, causing decreased nitric oxide, increased factors involved in coagulation or fibrinolysis such as plasminogen activating inhibitor-1 (PAI-1), tissue factor (TF), tissue plasminogen activator (tPA), increased adhesion molecules such as ICAM and VCAM, increased growth factors such as bFGF, TGFb, PDGF, VEGF, all factors causing cell growth inflammation and fibrosis.

The treatment e.g. of endothelial dysfunction can be demonstrated in the following pharmacological test:

### Material and methods

Male 20-24 week-old SHR, purchased from RCC Ldt (Fullingsdorf, Switzerland), are maintained in a temperature- and light-controlled room with free access to rat chow (Nafag 9331, Gossau, Switzerland) and tap water. The experiment is performed in accordance with the NIH guidelines and approved by the Canton Veterinary office (Bew 161, Kantonales Veterinäramt. Liestal, Switzerland). All rats are treated with the NO synthesis inhibitor L-NAME (Sigma Chemicals) administered in drinking water (50 mg/l) for 12 weeks. The average daily dose of L-NAME calculated from the water consumed was 2.5 mg/kg/d (range 2.1-2.7).

The rats can be divided into 5 groups: group 1, control (n = 40); Group 2, valsartan (val; n = 40); Group 3, amiloride (ami; n = 30); Group 4, hydrochlorothiazide (HCTZ; n = 30); Group 4, a combination (val-ami-HCTZ) (n = 30). The drugs are administered in drinking fluid. The doses to be used are selected from the work of Sweet et al. (1987) indicating significantly increased survival in rats with healed myocardial infarction. The pressor effect of Ang II at 1 mg/kg obtained in controls normotensive rats can be reducted after treatment with the compound of formula (I) in form of the hemi-fumarate (Gervais et al. 1999).

Body weight is measured every week. Systolic blood pressure and heart rate are recorded by tail cuff plethysmography 3 and 2 weeks before starting the study and at 2 weeks after drug administration. Urine is collected over a 24 hour period from rats kept in individual (metabolic) cages the week before starting treatment and at weeks 4 and 12 for volume measurement and protein, creatinine, sodium and potassium determination using standard laboratory methods. At the same time points, blood samples are withdrawn from the retro-orbital plexus (maximum 1 ml) for creatinine, Na⁺ and K⁺ assays.

Ten rats from each group are sacrificed at 4 weeks for collection of kidney and heart for morphological analysis. The remaining rats are sacrificed at 12 weeks. Cardiac and kidney weight is recorded. Terminal blood sampling is performed in 5 % EDTA at 4 (morphometry study) and 12 (end of the study) weeks for aldosterone, determination by radioimmunoassay using a DPC coat-a-count aldosterone-RIA kit (Bühlmann, Switzerland).

### Statistical analysis:

All data are expressed as mean ± SEM. Statistical analysis is performed using a one-way ANOVA, followed by a Duncan's multiple range test and a Newman-Keuls test, 7 for comparison between the different groups. Results with a probability value of less than 0.05 are deemed statistically significant.

### Results:

Even at non-blood pressure reducing doses, administration of the combination of the present invention leads to significant improvements in survival rates.

An improvement of regression of artherosclerosis without effecting the serum lipid levels can, for exmple, be demonstrated by using the animal model as disclosed by H. Kano et al. in Biochemical and Biophysical Research Communications 259, 414-419 (1999).

That the compounds or combinations according to the present invention can be used for the regression of a cholesterol diet-induced atherosclerosis, can be demonstrated using the test model described, e.g., by C. Jiang et al. in Br. J. Pharmacol. (1991), 104, 1033-1037.

Further benefits when applying the composition of the present invention are that lower doses of the individual drugs to be combined according to the present invention can be used to reduce the dosage, for example, that the dosages need not only often be smaller but are also applied less frequently, or can be used in order to diminish the incidence of side effects. This is in accordance with the desires and requirements of the patients to be treated.

### Design of Clinical Programs

A factorial design study in naïve or previously treated hypertensive patients is initated in order to select the more appropriate dose(s) for subsequent use will be. The positive outcome of the selected dosage is based on synergy in blood pressure lowering, low incidence of side effects and better potassium handling of the combination. This study includes up to 120 patients in every cells of the explored doses of monotherapy and/or marketed combination of two of the three components of the triple combination.

Furthermore, non-responder study is carried out to show that the add on of a third agent of the combination may bring additional blood pressure lowering and more patients under control without increasing the side effects.
These study(s) show that this triple combination provides additional myocardial protection in patients having myocardial infarction, acute coronary syndrome, ischemic heart disease, myocardial revascularization at the acute or chronic phase of coronary occlusion. This claim is supported by clinical studies measuring markers of myocardial ischemia and injury such as ejection fraction, left ventricular dimensions and contractility measured by MRI, echography, scintigraphy etc. In addition measurement of myocardial salvage by technetium scintigraphy or other appropriate measure of myocardial salvage.

Preferably, the jointly therapeutically effective amounts of the active agents according to the combination of the present invention can be administered simultaneously or sequentially in any order, separately or in a fixed combination.

The pharmaceutical composition according to the present invention as described hereinbefore and hereinafter may be used for simultaneous use or sequential use in any order, for separate use or as a fixed combination.

Furthermore, the present invention relates to the use of a combination comprising
((i) valsartan or a pharmaceutically acceptable salt thereof, and
(ii) amiloride or triameterine or a pharmaceutically acceptable salt thereof and
(iii) a further diuretic or a pharmaceutically acceptable salt thereof;
for the manufacture of a medicament for the prevention of, delay of progression of, or treatment of a disease or condition selected from the group consisting of
(a) hypertension, congestive heart failure, restenosis after percutaneous transluminal angioplasty, and restenosis after coronary artery bypass surgery;
(b) atherosclerosis, insulin resistance and syndrome X, diabetes mellitus type 2, obesity, nephropathy, hypothyroidism survival post myocardial infarction (MI), coronary heart diseases, hypertension in the elderly, familial dyslipidemic hypertension, increase of formation of collagen, fibrosis, and remodeling following hypertension (antiproliferative effect of the combination), all these diseases or conditions associated with or without hypertension;
(c) endothelial dysfunction with or without hypertension;
(d) hyperlipidemia, hyperlipoproteinemia, atherosclerosis and hypercholesterolemia;
(e) glaucoma; furthermore
(f) isolated systolic hypertension (ISH),
(g) diabetic retinopathy, and
(h) peripheral vascular disease.

The invention furthermore relates to a pharmaceutical composition for the prevention of, delay of progression of, treatment of a disease or condition selected from
(a) hypertension, congestive heart failure, restenosis after percutaneous transluminal angioplasty, and restenosis after coronary artery bypass surgery;
(b) atherosclerosis, insulin resistance and syndrome X, diabetes mellitus type 2, obesity, nephropathy, hypothyroidism, survival post myocardial infarction (MI), coronary heart diseases, hypertension in the elderly, familial dyslipidemic hypertension, increase of formation of collagen, fibrosis, and remodeling following hypertension (antiproliferative effect of the combination), all these diseases or conditions associated with or without hypertension;
(c) endothelial dysfunction with or without hypertension;
(d) hyperlipidemia, hyperlipoproteinemia, atherosclerosis and hypercholesterolemia;
(e) glaucoma; furthermore
(f) isolated systolic hypertension (ISH),
(g) diabetic retinopathy, and
(h) peripheral vascular disease;
comprising
((i) valsartan ora pharmaceutically acceptable salt thereof, and
(ii) amiloride or triameterine or a pharmaceutically acceptable salt thereof and
(iii) a further diuretic or a pharmaceutically acceptable salt thereof;
and a pharmaceutically acceptable carrier.

Further benefits when applying the composition of the present invention are that lower doses of the individual drugs to be combined according to the present invention can be used to reduce the dosage, for example, that the dosages need not only often be smaller but are also applied less frequently, or can be used in order to diminish the incidence of side effects. This is in accordance with the desires and requirements of the patients to be treated.

Preferably, the jointly therapeutically effective amounts of the active agents according to the combination of the present invention can be administered simultaneously or sequentially in any order, separately or in a fixed combination.

The pharmaceutical composition according to the present invention as described hereinbefore and hereinafter may be used for simultaneous use or sequential use in any order, for separate use or as a fixed combination.

These pharmaceutical preparations are for enteral, such as oral, and also rectal or parenteral, administration to homeotherms, with the preparations comprising the pharmacological active compound either alone or together with customary pharmaceutical auxiliary substances. For example, the pharmaceutical preparations consist of from about 0.1 % to 90 %, preferably of from about 1 % to about 80 %, of the active compound. Pharmaceutical preparations for enteral or parenteral, and also for ocular, administration are, for example, in unit dose forms, such as coated tablets, tablets, capsules or suppositories and also ampoules. These are prepared in a manner that is known per se, for example using conventional mixing, granulation, coating, solubulizing or lyophilizing processes. Thus, pharmaceutical preparations for oral use can be obtained by combining the active compound with solid excipients, if desired granulating a mixture which has been obtained, and, if required or necessary, processing the mixture or granulate into tablets or coated tablet cores after having added suitable auxiliary substances.

The dosage of the active compound can depend on a variety of factors, such as mode of administration, homeothermic species, age and/or individual condition.

Preferred dosages for the active ingredients of the pharmaceutical combination according to the present invention are therapeutically effective dosages, especially those which are commerically available.

Normally, in the case of oral administration, an approximate daily dose of from 1 mg to 360 mg is to be estimated e.g. for a patient of approximately 75 kg in weight.

The dosage of the active compound can depend on a variety of factors, such as mode of administration, homeothermic species, age and/or individual condition.

The pharmaceutical preparation will be supplied in the form of suitable dosage unit form, for example, a capsule or tablet for oral treatment.

Valsartan, as a representative of the class of AT₁-receptor antagonists, will be supplied in the form of suitable dosage unit form, for example, a capsule or tablet, and comprising a therapeutically effective amount, e.g. from 20 to 320 mg, of valsartan which may be applied to patients. The application of the active ingredient may occur up to three times a day, starting e.g. with a daily dose of 20 mg or 40 mg of valsartan, increasing via 80 mg daily and further to 160 mg daily up to 320 mg daily. Preferably, valsartan is applied twice a day with a dose of 80 mg or 160 mg, respectively, each. Corresponding doses may be taken, for example, in the morning, at mid-day or in the evening. Preferred is b.i.d administration.

Hydrochlorothiazide will be supplied in of suitable dosage unit form, for example, a capsule or tablet, and comprising a therapeutically effective amount, e.g. from 5 mg to 50 mg which may be applied to patients. Preferred doses per unit dosage form is 6,25 mg, 12,5 mg or 25 mg. The application of the active ingredient may occur up to three times a day.

The dosage of amiloride or triameterine, respectively, are those that are normally being used for mono-therapy, most preferably, the lower range of the prescribed doses. The application of the active ingredient may occur up to three times a day.

Especially preferred are low dose combinations

The following examples illustrate the above-described invention:
amiloride hydrochloride, and (iii) a dose of hydrochlorothiazide selected from 12.5 and 25 mg of hydrochlorothiazide.

Preferred are dosage unit forms or a single dosage unit form comprising (i) a dose of valsartan selected from 40 mg, 80 mg, 160 mg and 320 mg of valsartan, (ii) 5 mg of amiloride hydrochloride, and (iii) a dose of hydrochlorothiazide selected from 12.5 and 25 mg of hydrochlorothiazide.

Especially preferred are low dose combinations.

The following examples illustrate the above-described invention.

### Formulation Example 1:

### Film-Coated Tablets:

| Components | Composition Per Unit (mg) | Standards |
|---|---|---|
| Granulation | | |
| Valsartan [= active ingredient] | 80.00 | |
| Microcrystalline cellulose/ Avicel PH 102 | 54.00 | NF, Ph. Eur |
| Crospovidone | 20.00 | NF, Ph. Eur |
| Colloidal anhydrous silica / colloidal silicon dioxide Aerosil 200 | 0.75 | Ph. Eur/NF |
| Magnesium stearate | 2.5 | NF, Ph. Eur |

| Blending | | |
|---|---|---|
| Colloidal anhydrous silica / colloidal silicon dioxide / Aerosil 200 | 0.75 | Ph. Eur/NF |
| Magnesium stearate | 2.00 | NF, Ph. Eur |

| Coating | | |
|---|---|---|
| Purified water^{*)} | | |
| DIOLACK pale red 00F34899 | 7.00 | |
| Total tablet mass | 167.00 | |

| | | |
|---|---|---|
| ^{*)} Removed during processing. | | |

The film-coated tablet is manufactured e.g. as follows:

A mixture of valsartan, microcrystalline cellulose, crospovidone, part of the colloidal anhydrous silica/colloidal silicon dioxide/Aerosile 200, silicon dioxide and magnesium stearate is premixed in a diffusion mixer and then sieve through a screnning mill. The resulting mixture is again pre-mixed in a diffusion mixer, compacted in a roller compacter and then sieve through a screening mill. To the resulting mixture, the rest of the colloidal anhydrous silica/colloidal silicon dioxide/Aerosile 200 are added and the final blend is made in a diffusion mixer. The whole mixture is compressed in a rotary tabletting machine and the tabletts are coated with a film by using Diolack pale red in a perforated pan.

### Formulation Example 2:

### Film-coated tablets:

| Components | Composition Per Unit (mg) | Standards |
|---|---|---|
| Granulation | | |
| Valsartan [= active ingredient] | 160.00 | |
| Microcrystalline cellulose/ Avicel PH 102 | 108.00 | NF, Ph. Eur |
| Crospovidone | 40.00 | NF, Ph. Eur |
| Colloidal anhydrous silica / colloidal silicon dioxide Aerosil 200 | 1.50 | Ph. Eur/NF |
| Magnesium stearate | 5.00 | NF, Ph. Eur |

| Blending | | |
|---|---|---|
| Colloidal anhydrous silica / colloidal silicon dioxide / Aerosil 200 | 1.50 | Ph. Eur/NF |
| Magnesium stearate | 4.00 | NF, Ph. Eur |

| Coating | | |
|---|---|---|
| Opadry Light Brown 00F33172 | 10.00 | |
| Total tablet mass | 330.00 | |

The film-coated tablet is manufactured e.g. as described in Formulation Example 1.

### Formulation Example 3:

### Film-Coated Tablets:

| Components | Composition Per Unit (mg) | Standards |
|---|---|---|
| Core Internal phase | | |
| Valsartan | 40.00 | |
| [= active ingredient] | | |
| Silica, colloidal anhydrous (Colloidal silicon dioxide) | 1.00 | Ph. Eur, USP/NF |
| [= Glidant] | | |
| Magnesium stearate | 2.00 | USP/NF |
| [= Lubricant] | | |
| Crospovidone | 20.00 | Ph. Eur |
| [Disintegrant] | | |
| Microcrystalline cellulose | 124.00 | USP/NF |
| [= Binding agent] | | |

| External phase | | |
|---|---|---|
| Silica, colloidal anhydrous, (Colloidal silicon dioxide) | 1.00 | Ph. Eur, USP/NF |
| [= Glidant] | | |
| Magnesium stearate | 2.00 | USP/NF |
| [Lubricant] | | |

| Film coating | | |
|---|---|---|
| Opadry^{®} brown OOF 16711^{*)} | 9.40 | |
| Purified Water^{**)} | | |
| Total mass | 199.44 | |

| | | |
|---|---|---|
| ^{*)} The composition of the Opadry^{®} brown OOF16711 coloring agent is tabulated below. ^{**)} Removed during processing | | |

### Opadry^{®} Composition:

| Ingredient | Approximate % Composition |
|---|---|
| Iron oxide, black (C.I. No. 77499, E 172) | 0.50 |
| Iron oxide, brown (C.I. No. 77499, E 172 | 0.50 |
| Iron oxide, red (C.I. No. 77491, E 172) | 0.50 |
| Iron oxide, yellow (C.I. No. 77492, E 172) | 0.50 |
| Macrogolum (Ph. Eur) | 4.00 |
| Titanium dioxide (C.I. No. 77891, E 171) | 14.00 |
| Hypromellose (Ph. Eur) | 80.00 |

The film-coated tablet is manufactured e.g. as described in Formulation Example 1.

### Formulation Example 4:

### Capsules:

| Components | Composition Per Unit (mg) |
|---|---|
| Valsartan [= active ingredient] | 80.00 |
| Microcrystalline cellulose | 25.10 |
| Crospovidone | 13.00 |
| Povidone | 12.50 |
| Magnesium stearate | 1.30 |
| Sodium lauryl sulphate | 0.60 |

| Shell | |
|---|---|
| Iron oxide, red | 0.123 |
| (C.I. No. 77491, EC No. E 172) | |
| Iron oxide, yellow | 0.123 |
| (C.I. No. 77492, EC No. E 172) | |
| Iron oxide, black | 0.245 |
| (C.I No. 77499, EC No. E 172) | |
| Titanium dioxide | 1.540 |
| Gelatin | 74.969 |
| Total tablet mass | 209.50 |

The tablet is manufactured e.g. as follows:
Granulation/Drying
   Valsartan and microcrystallin cellulose are spray-granulated in a fluidised bed granulator with a granulating solution consisting of povidone and sodium lauryl sulphate dissolved in purified water. The granulate obtained is dried in a fluidiesd bed dryer.
Milling/Blending
   The dried granulate is milled together with crospovidone and magnesium stearate. The mass is then blended in a conical srew type mixer for approximately 10 minutes.
Encapsulation
   Teh empty hard gelatin capsules are filled with the blended bulk granules under controlled temperature and humidity conditions. The filed capsules are dedustee, visually inspected, weightchecked and quarantied until by Quality assurance department.

### Formulation Example 5:

### Capsules:

| Components | Composition Per Unit (mg) |
|---|---|
| Valsartan [= active ingredient] | 160.00 |
| Microcrystalline cellulose | 50.20 |
| Crospovidone | 26.00 |
| Povidone | 25.00 |
| Magnesium stearate | 2.60 |
| Sodium lauryl sulphate | 1.20 |

| Shell | |
|---|---|
| Iron oxide, red (C.I. No. 77491, EC No. E 172) | 0.123 |
| Iron oxide, yellow | 0.123 |
| (C.I. No. 77492, EC No. E 172) | |
| Iron oxide, black | 0.245 |
| (C.I. No. 77499, EC No. E 172) | |
| Titanium dioxide | 1.540 |
| Gelatin | 74.969 |
| Total tablet mass | 342.00 |

The formulation is manufactured e.g. as described in Formulation Example 4.

### Formulation Example 6:

### Hard Gelatine Capsule:

| ComponentS | Composition Per Unit (mg) |
|---|---|
| Valsartan [= active ingredient] | 80.00 |
| Sodium laurylsulphate | 0.60 |
| Magnesium stearate | 1.30 |
| Povidone | 12.50 |
| Crospovidone | 13.00 |
| Microcrystalline cellulose | 21.10 |
| Total tablet mass | 130. 00 |

### Examples 7 to 11:

| Example | 7 | 8 | 9 | 10 | 11 |
|---|---|---|---|---|---|
| Components | **Composition per unit (mg)** | **Composition per unit (mg)** | **Composition per unit (mg)** | **Composition per unit (mg)** | **Composition per unit (mg)** |
| **Granulation** | | | | | |
| Valsartan Drug Substance (DS) | 80.000 | 160.000 | 40.000 | 320.000 | 320.000 |
| Microcrystalline Cellulose (NF, Ph.Eur.)/ Avicel PH 102 | 54.000 | 108.000 | 27.000 | 216.000 | 216.000 |
| Crospovidone (NF, Ph.Eur.) | 15.000 | 30.000 | 7.500 | 80.000 | 60.000 |
| Colloidal Anhydrous Silica (Ph. Eur.)/Colloidal Silicon Dioxide (NF)/Aerosil 200 | 1.500 | 3.000 | 0.750 | 3.000 | 6.000 |
| Magnesium Stearate (NF, Ph.Eur.) | 3.000 | 6.000 | 1.500 | 10.000 | 12.000 |

| **Blending** | | | | | |
|---|---|---|---|---|---|
| Colloidal Anhydrous Silica (Ph. Eur.)/Colloidal Silicon Dioxide (NF)/Aerosil 200 | --- | --- | --- | 3.000 | - |
| Magnesium Stearate, NF, Ph.Eur. | 1.500 | 3.000 | 0.750 | 8.000 | 6.000 |
| **Core Weight/mg** | 155.000 | 310.000 | 77.500 | 640.000 | 620.000 |
| **Coating** | - | - | 3.800 | 15.000 | 16.000 |

## Claims

1. A combination, such as a combined preparation or pharmaceutical composition, respectively, comprising
(i) valsartan or a pharmaceutically acceptable salt thereof, and
(ii) the diuretic amiloride or triameterine or a pharmaceutically acceptable salt thereof, and
(iii) a further diuretic or a pharmaceutically acceptable salt thereof.

2. A combination according to claim 1, comprising
(i) valsartan or a pharmaceutically acceptable salt thereof and
(ii) amiloride or a pharmaceutically acceptable salt thereof and
(iii) hydrochlorothiazide or a pharmaceutically acceptable salt thereof.

3. A combination according to claim 1 comprising
(i) valsartan and
(ii) amiloride hydrochloride and
(iii) hydrochlorothiazide.

4. A pharmaceutical composition comprising a combination according to any one of claims 1 to 3.

5. A pharmaceutical composition for the prevention of, delay of progression of, treatment of a disease or condition selected from
(a) hypertension, congestive heart failure, restenosis after percutaneous transluminal angioplasty, and restenosis after coronary artery bypass surgery;
(b) atherosclerosis, insulin resistance and syndrome X, diabetes mellitus type 2, obesity, nephropathy, hypothyroidism, survival post myocardial infarction (MI), coronary heart diseases, hypertension in the elderly, familial dyslipidemic hypertension, increase of formation of collagen, fibrosis, and remodeling following hypertension (antiproliferative effect of the combination), all these diseases or conditions associated with or without hypertension;
(c) endothelial dysfunction with or without hypertension,
(d) hyperlipidemia, hyperlipoproteinemia, atherosclerosis and hypercholesterolemia, (e) glaucoma; furthermore
(f) isolated systolic hypertension (ISH),
(g) diabetic retinopathy, and
(h) peripheral vascular disease;
comprising
(i) valsartan or a pharmaceutically acceptable salt thereof, and
(ii) the diuretic amiloride or triameterine or a pharmaceutically acceptable salt thereof, and
(iii) a further diuretic or a pharmaceutically acceptable salt thereof; and
(iv) an auxiliary substance.

6. Use of a combination comprising
(i) valsartan or a pharmaceutically acceptable salt thereof, and
(ii) the diuretic amiloride or triameterine or a pharmaceutically acceptable salt thereof, and
(iii) a further diuretic or a pharmaceutically acceptable salt thereof;
for the manufacture of a medicament for the prevention of, delay of progression of, treatment of a disease or condition selected from
(a) hypertension, congestive heart failure, restenosis after percutaneous transluminal angioplasty, and restenosis after coronary artery bypass surgery;
(b) atherosclerosis, insulin resistance and syndrome X, diabetes mellitus type 2, obesity, nephropathy, hypothyroidism, survival post myocardial infarction (MI), coronary heart diseases, hypertension In the elderly, familial dyslipidemic hypertension, increase of formation of collagen, fibrosis, and remodeling following hypertension (antiproliferative effect of the combination), all these diseases or conditions associated with or without hypertension;
(c) endothelial dysfunction with or without hypertension,
(d) hyperlipidemia, hyperlipoproteinemia, atherosclerosis and hypercholesterolemia, (e) glaucoma; furthermore
(f) isolated systolic hypertension (ISH),
(g) diabetic retinopathy, and
(h) peripheral vascular disease.

## Patentansprüche

1. Kombination, wie ein Kombinationspräparat oder eine pharmazeutische Zusammensetzung, umfassend
(i) Valsartan oder ein pharmazeutisch akzeptables Salz hiervon, und
(ii) das Diuretikum Amilorid oder Triameterin oder ein pharmazeutisch akzeptables Salz hiervon, und
(iii) ein weiteres Diuretikum oder ein pharmazeutisch akzeptables Salz hiervon.

2. Kombination nach Anspruch 1, umfassend
(i) Valsartan oder ein pharmazeutisch akzeptables Salz hiervon, und
(ii) Amilorid oder ein pharmazeutisch akzeptables Salz hiervon, und
(iii) Hydrochlorthiazid oder ein pharmazeutisch akzeptables Salz hiervon.

3. Kombination nach Anspruch 1, umfassend
(i) Valsartan, und
(ii) Amiloridhydrochlorid und
(iii) Hydrochlorthiazid.

4. Pharmazeutische Zusammensetzung, umfassend eine Kombination nach einem der Ansprüche 1 bis 3.

5. Pharmazeutische Zusammensetzung zur Prävention, Progressionsverzögerung oder Behandlung einer Krankheit oder eines Zustands, ausgewählt aus
(a) Hypertension, kongestivem Herzversagen, Restenose nach einer perkutanen transluminalen Angioplastie und Restenose nach einer koronaren Arterienbypassoperation,
(b) Atherosklerose, Insulinresistenz und Syndrom X, Diabetes mellitus Typ 2, Obesität, Nephropathie, Hypothyroidismus, Überleben nach einem Myokardinfarkt (MI), koronaren Herzkrankheiten, Hypertension bei älteren Menschen, familiärer dyslipidämischer Hypertension, Erhöhung der Bildung von Collagen, Fibrose und Remodellierung nach Hypertension (antiproliferativer Effekt der Kombination), wobei all diese Krankheiten oder Zustände assoziiert sind mit oder ohne Hypertension,
(c) Endotheldysfunktion mit oder ohne Hypertension,
(d) Hyperlipidämie, Hyperlipoproteinämie, Atherosklerose und Hypercholesterolämie,
(e) Glaukom, ferner
(f) isolierter systolischer Hypertension (ISH),
(g) diabetischer Retinopathie und
(h) peripherer Vaskularkrankheit,
umfassend
(i) Valsartan oder ein pharmazeutisch akzeptables Salz hiervon, und
(ii) das Diuretikum Amilorid oder Triameterin oder ein pharmazeutisch akzeptables Salz hiervon, und
(iii) ein weiteres Diuretikum oder ein pharmazeutisch akzeptables Salz hiervon, und
(iv) einen Hilfsstoff.

6. Verwendung einer Kombination, umfassend
(i) Valsartan oder ein pharmazeutisch akzeptables Salz hiervon, und
(ii) das Diuretikum Amilorid oder Triameterin oder ein pharmazeutisch akzeptables Salz hiervon, und
(iii) ein weiteres Diuretikum oder ein pharmazeutisch akzeptables Salz hiervon,
zur Herstellung eines Arzneimittels für die Prävention, Progressionsverzögerung oder Behandlung einer Krankheit oder eines Zustands, ausgewählt aus
(a) Hypertension, kongestivem Herzversagen, Restenose nach einer perkutanen transluminalen Angioplastie und Restenose nach einer koronaren Arterienbypassoperation,
(b) Atherosklerose, Insulinresistenz und Syndrom X, Diabetes mellitus Typ 2, Obesität, Nephropathie, Hypothyroidismus, Überleben nach einem Myokardinfarkt (MI), koronaren Herzkrankheiten, Hypertension bei älteren Menschen, familiärer dyslipidämischer Hypertension, Erhöhung der Bildung von Collagen, Fibrose und Remodellierung nach Hypertension (antiproliferativer Effekt der Kombination), wobei all diese Krankheiten oder Zustände assoziiert sind mit oder ohne Hypertension,
(c) Endotheldysfunktion mit oder ohne Hypertension,
(d) Hyperlipidämie, Hyperlipoproteinämie, Atherosklerose und Hypercholesterolämie,
(e) Glaukom, ferner
(f) isolierter systolischer Hypertension (ISH),
(g) diabetischer Retinopathie und
(h) peripherer Vaskularkrankheit,

## Revendications

1. Combinaison telle qu'une préparation combinée ou composition pharmaceutique, comprenant respectivement
(i) du valsartan ou un de ses sels pharmaceutiquement acceptables, et
(ii) le diurétique amiloride ou triamtérène ou un de leurs sels pharmaceutiquement acceptables, et
(iii) un autre diurétique ou un de ses sels pharmaceutiquement acceptables.

2. Combinaison selon la revendication 1 comprenant
(i) du valsartan ou un de ses sels pharmaceutiquement acceptables, et
(ii) de l'amiloride ou un de ses sels pharmaceutiquement acceptables, et
(iii) de l'hydrochlorothiazide ou un de ses sels pharmaceutiquement acceptables.

3. Combinaison selon la revendication 1 comprenant
(i) du valsartan et
(ii) du chlorhydrate d'amiloride et
(iii) de l'hydrochlorothiazide.

4. Composition pharmaceutique comprenant une combinaison selon l'une quelconque des revendications 1 à 3.

5. Composition pharmaceutique destinée à la prévention, au ralentissement de l'évolution, au traitement d'une maladie ou d'une affection choisie parmi
(a) l'hypertension, l'insuffisance cardiaque congestive, la resténose après une angioplastie transluminale percutanée, et la resténose après une chirurgie de pontage aorto-coronaire ;
(b) l'athérosclérose, l'insulinorésistance et le syndrome X, le diabète sucré de type 2, l'obésité, la néphropathie, l'hypothyroïdie, la survie après un infarctus du myocarde (IM), les maladies coronariennes, l'hypertension du sujet âgé, l'hypertension familiale dyslipidémique, l'augmentation de la formation de collagène, la fibrose, et le remodelage résultant de l'hypertension (effet antiprolifératif de la combinaison), toutes ces maladies ou affections étant associées ou non à une hypertension ; et
(c) le dysfonctionnement endothélial avec ou sans hypertension,
(d) l'hyperlipidémie, l'hyperlipoprotéinémie, l'athérosclérose et l'hypercholestérolémie,
(e) le glaucome ; ainsi que
(f) l'hypertension systolique isolée (HSI),
(g) la rétinopathie diabétique, et
(h) la maladie vasculaire périphérique ;
comprenant
(i) du valsartan ou un de ses sels pharmaceutiquement acceptables, et
(ii) le diurétique amiloride ou triamtérène ou un de leurs sels pharmaceutiquement acceptables, et
(iii) un autre diurétique ou un de ses sels pharmaceutiquement acceptables ; et
(iv) une substance auxiliaire.

6. Utilisation d'une combinaison comprenant
(i) du valsartan ou un de ses sels pharmaceutiquement acceptables, et
(ii) le diurétique amiloride ou triamtérène ou un de leurs sels pharmaceutiquement acceptables, et
(iii) un autre diurétique ou un de ses sels pharmaceutiquement acceptables ;
pour la fabrication d'un médicament destiné à la prévention, au ralentissement de l'évolution, au traitement d'une maladie ou d'une affection choisie parmi
(a) l'hypertension, l'insuffisance cardiaque congestive, la resténose après une angioplastie transluminale percutanée, et la resténose après une chirurgie de pontage aorto-coronaire ;
(b) l'athérosclérose, l'insulinorésistance et le syndrome X, le diabète sucré de type 2, l'obésité, la néphropathie, l'hypothyroïdie, la survie après un infarctus du myocarde (IM), les maladies coronariennes, l'hypertension du sujet âgé, l'hypertension familiale dyslipidémique, l'augmentation de la formation de collagène, la fibrose, et le remodelage résultant de l'hypertension (effet antiprolifératif de la combinaison), toutes ces maladies ou affections étant associées ou non à une hypertension ; et
(c) le dysfonctionnement endothélial avec ou sans hypertension,
(d) l'hyperlipidémie, l'hyperlipoprotéinémie, l'athérosclérose et l'hypercholestérolémie,
(e) le glaucome ; ainsi que
(f) l'hypertension systolique isolée (HSI),
(g) la rétinopathie diabétique, et
(h) la maladie vasculaire périphérique.
